# EUROPEAN PATENT SPECIFICATION

(11) **EP 1 801 587 B1**
(45) Date of publication and mention of the grant of the patent: **05.12.2012**
(21) Application number: 06020882.4
(22) Date of filing: 04.10.2006
(51) Int. Cl.: G01N 33/50

(54) **Method of detecting a biopolymer and device for the same**
Verfahren zur Bestimmung von Biopolymeren und Vorrichtung dafür
Procédé et dispositif de détection d'un biopolymère

(30) Priority: 05.10.2005 JP 2005292518
(43) Date of publication of application: 27.06.2007
(73) Proprietor: Hitachi Solutions, Ltd., Tokyo 140-0002 (JP)
(72) Inventor: SATO, Keiichi, Tokyo 140-0002 (JP)
(74) Representative: Liesegang, Roland

(56) References cited:
- EP-A- 0 044 140
- US-A1- 2002 173 493
- LAZAROVA Z ET AL: "Integrated reversed micellar extraction and stripping of [alpha]-amylase" BIOTECHNOLOGY AND BIOENGINEERING 05 JUN 1999 UNITED STATES, vol. 63, no. 5, 5 June 1999 (1999-06-05), pages 583-592, XP002433237 ISSN: 0006-3592
- BHATTACHARYA S ET AL: "Interaction of surfactants with DNA. Role of hydrophobicity and surface charge on intercalation and DNA melting" BIOCHIMICA ET BIOPHYSICA ACTA, AMSTERDAM, NL, vol. 1323, no. 1, 14 January 1997 (1997-01-14), pages 29-44, XP002410344 ISSN: 0006-3002

## Description

### BACKGROUND OF THE INVENTION

### Field of the Invention

The present invention relates to a method of quickly and simply detecting and measuring a biopolymer such as DNA. Also disclosed is a device used for the detection and measurement.

### Description of the Prior Art

Conventionally, in the field of detection and measurement of biopolymers, a DNA chip technique, in which the detection and measurement are performed by fixing a plurality of probe DNAs having known characteristics on a flat substrate, and a flow cytometry technique, which utilizes beads each having a biopolymer with known characteristics fixed thereon, have so far been used. These techniques are very useful to collectively obtain a large amount of information on a tested biopolymer. Moreover, in recent years, the study has particularly been advanced. As a result of that, necessary information can be obtained even with a small number of genes.

On the other hand, focusing on the fact that DNA possesses a negative electric charge, another method has been proposed. In this method, labeled DNA samples dissolved in a water phase is encapsulated by a cationic surfactant, thus forming a reverse micelle. The reverse micelle is taken into an organic phase, and accordingly, DNA is purified and concentrated. For example, J. Chem. Eng. Jpn. 37(5) pp. 662-668 (2004) describes a technique in which DNA concentration is performed by transferring DNA between phases by using ammonium salt as a cationic surfactant, and by utilizing the interaction between negative charge of the DNA and positive charge of the surfactant.

In addition, the present inventor et al. have made public and executed a method of precisely detecting and measuring a tested biopolymer as follows. After hybridizing biopolymer samples and probes with each other, by separating reacted samples and unreacted samples from each other, abundances thereof are measured (refer to Japanese Patent Applications 2002-168864 and 2002-228664). Furthermore, the present inventor et al. have made public and executed a technique for coating surfaces of semiconductor nanoparticles to provide resistance to change in pH thereto (refer to Japanese Patent Application 2005-103746).

EP 0 044 140 A1 discloses a method of carrying out a binding assay wherein an aqueous reaction mixture, after a binding interaction, is reacted with reactant which interacts preferentially with a labeled version of one or other of the binding reagents to give rise to a product having solubility properties which are substantially different from those of the original binding reagents or complex, and thereafter partitioning hydrophobic substances from the treated reaction mixture into a hydrophobic solvent phase and detecting the labeled material present in the hydrophobic phase.

In recent years, unnatural compounds such as PNA (Peptide Nucleic Acids) and LNA (Locked Nucleic Acids) have been synthesized, the unnatural compounds having a structure similar to that of DNA. Unlike DNA, the backbone of PNA is held together by peptide bonds instead of phosphate bonds. For this reason, unlike DNA, PNA does not carry negative charge. Moreover, PNA is more strongly hybridized with biopolymer samples, in comparison to DNA, and PNA thus has a higher ability to recognize complementary strand. As a result, it contributes to performance of a highly sensitive detection and measurement in a short period of time, and enables easy detection of single-base substitutions in the sequence thereof, thus being expected to be widely applied. LNA is a nucleic acid having two cyclic structures by a methylene bridge connecting an oxygen atom at 2' site with a carbon atom at 4' site of ribonucleoside, and LNA does not carry negative electric charge like DNA.

### SUMMARY OF THE INVENTION

In detection and measurement of a biopolymer by using the DNA chip technique or the flow cytometry technique, the preparation of samples is generally required as a pre-processing for the detection and measurement, such as the separation and the purification of a biopolymer to be tested, and the bonding of a label to the biopolymer. This preparation process is very complicated, and it is difficult to strictly perform the sample preparation. For this reason, it is difficult to determine the quantity of the sample biopolymers with high accuracy in the following detection and measurement processes. Additionally, it takes a certain length of time to perform the operation required before obtaining the results of detection and measurement after performing the sample preparation process. Moreover, a device for such an operation tends to be large-scaled and costly. Accordingly, there has been a problem that such techniques described above cannot be applied to some cases, for example, a case where it is required to urgently analyze of a blood sample in medical practice.

The present invention is made in consideration of the above situation. An object of the present invention is to provide a method of quickly and simply detecting and measuring a biopolymer with high accuracy, and a device for the detection and measurement. The present invention relates to a method of detecting a biopolymer utilising two kinds of solvent phases as outlined in the claims.

As a result of devoting themselves to study in consideration of the above problems to be solved, the present inventor et al. found out the fact that it becomes possible to simply and quickly detect and measure a biopolymer with high accuracy without using a special device by combining a technology in which the DNA samples described above are transferred from one phase to the other, thus separating and purifying after being encapsulated by use of a cationic surfactant with the using of similar compounds to DNA, namely PNA and LNA.

Specifically, the present invention provides a method of detecting a biopolymer utilizing two kinds of solvent phases having different properties from each other. The method includes: a step of allowing sample biopolymers to react with a labeled probe biopolymer in the first solvent phase; a step of transferring a product of the reaction of the sample biopolymers with the probe biopolymer to the second solvent phase using medium having surface reactivity between the two kinds of solvent phases; and a step of detecting the reaction product contained in the second solvent phase by use of the label, as outlined in the appended claims.

The present invention also provides a method of detecting a biopolymer utilizing two kinds of solvent phases having different properties from each other. The method includes steps of: allowing sample biopolymers to react with a labeled probe biopolymer in the first solvent phase; transferring a product of the reaction of the sample biopolymers with the probe biopolymers to the second solvent phase using medium having surface reactivity between the two kinds of solvent phases; and detecting the probe biopolymer contained in the first solvent phase, and the reaction product contained in the second solvent phase, respectively, by use of the label, as outlined in the appended claims. This method of detecting a biopolymer is further characterized in that the reactivity between the sample biopolymers and the probe biopolymer is determined by comparing the detected amount of the probe biopolymer contained in the first solvent phase with the detected amount of the reaction product contained in the second solvent phase.

In addition, the method of detecting a biopolymer according to the present invention is characterized as follows. The first solvent phase is a water phase and the above second solvent phase is an organic phase. The sample biopolymers have polarity and the probe biopolymer hardly has polarity. The medium having the surface activity contains a portion having polarity and a portion having little polarity.

In the method of detecting a biopolymer according to the present invention, the probe biopolymer is a molecule having no polarity, namely PNA and LNA. Moreover, the method is characterized in that the probe biopolymer is preferably labeled by a molecule or particle, such as a fluorescence material and a semiconductor nanoparticle. The method is further characterized in that the above medium having the surface activity is a cationic surfactant.

Also disclosed is a device for detecting sample biopolymers by the above-described method of detecting a biopolymer. The device includes the first solvent phase and the second solvent phase. The first solvent phase contains the labeled probe biopolymers, and the second solvent phase contains the medium having the surface activity between the above two kinds of solvent phases. This device for detecting a biopolymer is preferably composed of a closed container. Advantages of the Invention

As described above, the method of detecting a biopolymer according to the present invention and the device for the same allow a simple, quick, and highly accurate detection and measurement of the biopolymer without using a special device.

### BRIEF DESCRIPTION OF THE DRAWINGS

Fig. 1 is a view showing a step of allowing samples to react with probes in a method of detecting a biopolymer according to the present invention.
Fig. 2 shows the step of separating reacted samples in the method of detecting a biopolymer according to the present invention.
Fig. 3 shows a device used to simply carry out the method of detecting a biopolymer according to the present invention.

### DETAILED DESCRIPTION OF THE PREFERRED EMBODIMENTS

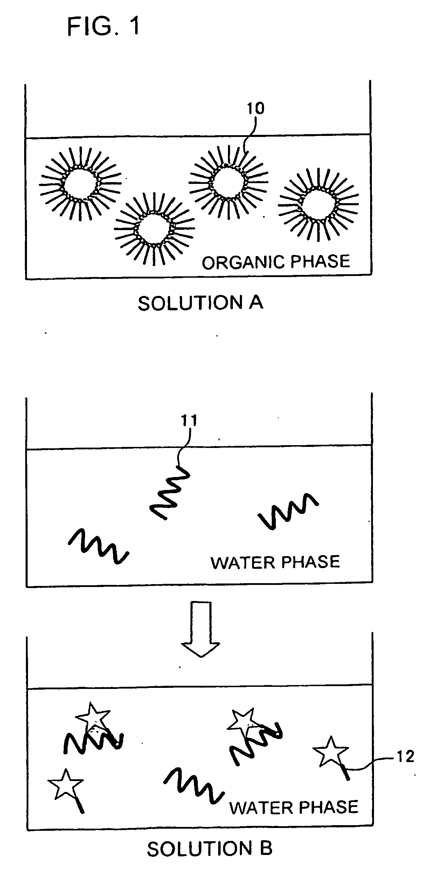

Hereinafter, referring to the accompanied drawings, the best mode for carrying out a method of detecting a biopolymer according to the present invention and a device for detecting the same is described in detail. Figs. 1 to 3 are views illustrating the embodiments of the present invention. In these drawings, parts assigned with identical symbols indicate the identical parts, and the basic construction and operation are identical.

In the embodiments shown below, PNA having a known sequence and DNA are used as probe biopolymers and as sample biopolymers. Also, ammonium salt is used as a cationic surfactant. The cationic surfactant preferably has 2 or 3 alkyl chains, and more preferably the carbon chain length of each of the alkyl chains of the cationic surfactant is 8 to 18. In addition, a pigment, a semiconductor nanoparticle, a complex, and the like, are used for labeling a probe PNA. Moreover, hexane, isooctane, chloroform, ethyl acetate, and the like, can also be used as a solvent of an organic phase.

Fig. 1 is a view showing a step at which the reaction of the samples is occurred in the method of detecting a biopolymer according to the present invention. First, a solution A (organic phase) is prepared by adding a cationic surfactant 10 composed of ammonium salt to an organic solvent. Concentration of the ammonium salt is not specifically limited because it varies depending on reaction conditions. However, the concentration thereof may be about several mM. On the other hand, a solution B (water phase) is prepared by dissolving sample DNAs 11 in a phosphate buffer solution. To the solution B, a labeled probe PNA 12 is added, so as to perform a hybridization reaction between the sample DNAs 11 and the probe PNA 12. At the time of the reaction, the reaction temperature, reaction time, the concentration and pH of the phosphate buffer solution are to be set to suitable values, respectively, for each sample to be treated. At this time, all of the sample DNAs I 1 are not necessarily allowed to react with the probe PNA 12. The solution B after the reaction possibly contains a reaction product of the sample and the probe (hereinafter referred to as a reacted sample), an unreacted sample, and an unreacted probe. Subsequently, the process is advanced to the next step of separating the reacted samples.

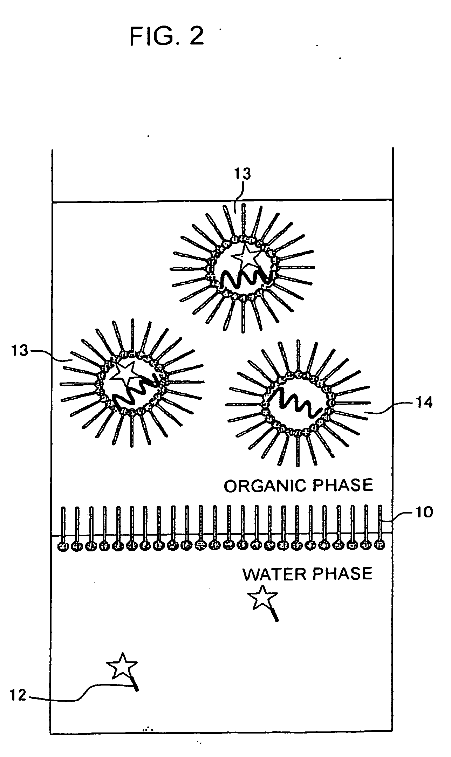

Fig. 2 is a view showing a step at which the reacted samples are separated in the method of detecting a biopolymer according to the present invention. When mixing the above solution A and the reacted solution B, the organic phase and the water phase are separated from each other. When centrifuging this mixed solution after vigorously agitating it, the organic phase and the water phase are again separated from each other. Regardless of whether or not the sample DNAs 11 in the water phase have been reacted with the probe PNA 12, this agitation causes the samples DNA 11 to electrostatically bond to a polar portion of the cationic surfactant 10, and to be extracted in the organic phase in a state of being encapsulated in a reverse micelle. Alcohol, and the like, may be added to the mixed solution in order to fasten this extraction. Meanwhile, the probe PNA 12 is not bonded to the cationic surfactant 10 because the probe PNA 12 has little polarity. Thus, almost all of the unreacted probe PNAs 12 stay in the water phase.

Then, as a step of detection and measurement, the organic phase and the water phase are separated from each other to measure abundance of the label in each phase. As shown in Fig. 2, the reacted samples 13 and the unreacted samples 14 are extracted in the organic phase. In the present invention, the probe PNA 12 is labeled, so the abundance of the label in the organic phase can directly be considered to be the abundance of the reacted samples 13 in the organic phase. On the other hand, the abundance of the label in the water phase can directly be considered to be the abundance of the unreacted probe PNAs 15 in the water phase. For this reason, the reactivity between the sample DNAs and the probe PNAs can be evaluated by making a relative comparison between the abundances of the label in the organic and in the water phases, respectively.

As described above, in the method of detecting a biopolymer according to the present invention, labeling is performed on the probe side unlike the conventional method in which labeling is performed on the sample side. For this reason, the only reacted samples can be detected and measured by using the label in the organic phase in which the reacted sample and the unreacted sample are intermingled. Thus, the present invention provides a detection method having an excellent quantitativity. Moreover, in actual detection and measurement, the only requirement is to prepare a probe which is previously labeled, thus, a process of labeling a sample can be eliminated, thus making it possible to achieve very convenient and quick detection and measurement.

In the method of detecting a biopolymer according to the present invention, for a reaction vessel, a reaction solution, probe, label, and experimental equipment, easily available ones can be used. Moreover, for the detection method, commonly used experimental processes such as agitation and centrifugation can be utilized. Therefore, detection and measurement can be performed easily and at low cost by utilizing existing equipment at an experimental laboratory, a medical care facility, and the like.

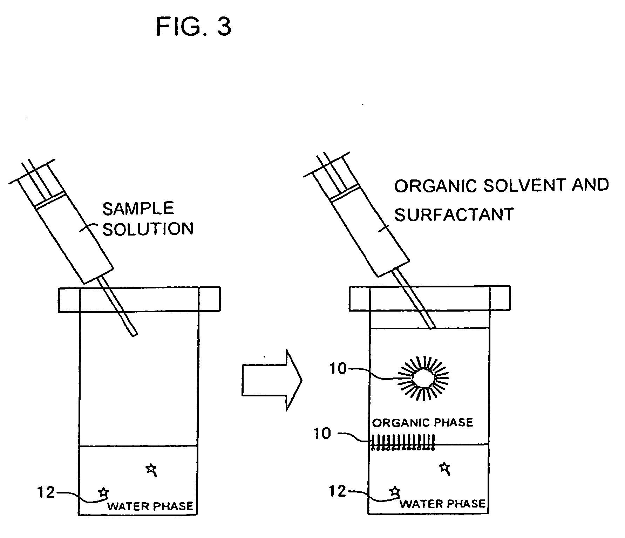

In order to simply carry out the method of detecting a biopolymer according to the present invention, equipment for detecting a biopolymer, as shown in Fig. 3, can be used. The equipment shown in Fig. 3 is a closed container containing an aqueous solution. The aqueous solution contains labeled probe PNA. Sample DNA is injected into a water phase in this container using an injection needle, and the like, followed by waiting for a certain time period to cause a reaction. Furthermore, an organic solution containing a cationic surfactant is injected into the container using an injection needle, and the like, followed by agitating and centrifuging. In this way, detection and measurement can be performed as the same manner as described above. Preparing such equipment for detecting a biopolymer in advance makes it possible to very quickly deal with a case where the urgent necessity to analyze a sample has occurred. Moreover, using such a closed container makes it possible to perform detection and measurement without exposing the sample to be measured, thus resulting in securing the safety of an operator.

As application of the method of detecting a biopolymer according to the present invention, it is also possible to obtain a large amount of information at once by increasing the number of the kind of the label. The label is not limited to a pigment and the like, and it does not matter what form it has, as long as it is possible to distinguish the reacted sample from the unreacted sample by using the label. For example, by modifying the probe with protein such as biotin or a functional group such as a carboxyl group, and using an amphipathic molecule having adivin, and the like, or amino group, and the like as a hydrophilic group, it is possible to enable the selective recognition of the probe molecule, thus achieving the object of the present invention. Furthermore, by using a substance which is visually recognizable as a label, another embodiment can be considered, in which there is no requirement of a measuring device. In the process of detecting and measuring the sample, detection and measurement with higher accuracy can also be performed, for example, by measuring the label in a state of generating clearer signal by allowing chemical reaction thereon instead of measuring the label as it is.

Description is above made of the method of detecting a biopolymer according to the present invention and device for the same by showing the specific embodiments. However, the present invention is not limited to these. Those skilled in the art can change and modify the constitutions and functions of the invention according to each of the above embodiments or another embodiment without departing from the scope of claim of the invention.

For example, both of the probe PNA and the sample DNA described above can be substituted by a biopolymer, such as DNA, PNA, LNA. Although, the labeling preferably is performed on the probe biopolymers, it may be possible to perform the labeling on the sample biopolymers. Alternatively, it is also conceivable to perform the labeling on both of them. The label of the probe may be, in addition to a fluorescence molecule, a substance which can be detected and measured by an electrochemical, a magnetic, or an optical method. An operator may use an arbitrary label with respect to experimental equipment to be used and an experimental purpose thereof. Furthermore, by using a molecule and a particle as a label, the molecule and the particle serving as a support which supports the probe biopolymers, and by selectively extracting the support, the same detection and measurement can be performed In this case, the sample biopolymers preferably are labeled. Moreover, as a surfactant, an ammonium salt is here used in the example. However, the surfactant is not limited to this because polarity and structure of a surfactant should be taken into consideration, based on the properties, and the like, of the sample and probe. The solvents used in pre-extraction and post-extraction phases are not limited to the substance shown above, and an arbitrary substance capable of achieving the object of the present invention may be used, on the basis of considering the combination of a sample, a probe, and a surfactant to be used. Regarding the labeling of the probe, by grouping the probe and performing labeling thereon, the measurement of a reaction can be performed on a group basis. In these cases, necessary results can be obtained by using only essential method and information. In particular, for the purposes of diagnosis and examination, efficient measurement and inspection can be performed. Also, measurement and detection can be performed without paying attention to the sample DNA into consideration. Industrial Applicability

The method of detecting a biopolymer according to the present invention and the device for the same used therein make it possible to simply and quickly carry out detection and measurement of a biopolymer sample in sites of gene analysis and medication, and the method is the industrially applicable invention.

## Claims

1. A method of detecting a biopolymer utilizing two kinds of solvent phases different in properties from each other, the method comprising the steps of:
allowing a sample biopolymer to react with a labeled probe biopolymer in a first solvent phase;
transferring a product of the reaction of the sample biopolymer with the probe biopolymer to a second solvent phase using a medium having surface activity between the two kinds of solvent phases by agitating a mixture of the first and second solvent phase, said first solvent phase being the solvent phase in which said sample biopolymer has been allowed to react with said labeled probe biopolymer; and
detecting the reaction product contained in the second solvent phase by use of the label,
wherein
the first solvent phase is a water phase,
the second solvent phase is an organic phase,
the sample biopolymer has polarity,
the probe biopolymer has little polarity, and
the medium having the surface activity contains a portion having polarity and a portion having little polarity, said medium being a cationic surfactant, and
wherein the probe biopolymer is any one of PNA (peptide nucleic acids) and LNA (locked nucleic acids), and wherein the sample biopolymer is DNA.

2. The method of detecting a biopolymer as set forth in claim 1, utilizing two kinds of solvent phases different in properties from each other, the method comprising the steps of:
allowing said sample biopolymer to react with said labeled probe biopolymer in said first solvent phase;
transferring said product of the reaction of the sample biopolymer with the probe biopolymer to said second solvent phase using said medium having surface activity between the two kinds of solvent phases; and
detecting the probe biopolymer contained in the first solvent phase, and the reaction product contained in the second solvent phase by use of the label.

3. The method of detecting a biopolymer as set forth in claim 2, wherein
reactivity between the sample biopolymer and the probe biopolymer is determined by comparing detected amount of the probe biopolymer contained in the first solvent phase with a detected amount of the reaction product contained in the second solvent phase.

4. The method of detecting a biopolymer as set forth in any of the claims 1-3, wherein the probe biopolymer is labeled by a particle,
wherein the particle is a semiconductor nanoparticle.

## Patentansprüche

1. Verfahren zum Nachweisen eines Biopolymers unter Verwendung von zwei Arten von Lösungsmittelphasen, die hinsichtlich ihrer Eigenschaften unterschiedlich zueinander sind, wobei das Verfahren die Schritte umfasst:
Reagierenlassen eines Proben-Biopolymers mit einem markierten Sonden-Biopolymer in einer ersten Lösungsmittelphase;
Übertragen eines Produkts der Reaktion des Proben-Biopolymers mit dem Sonden-Biopolymer in eine zweite Lösungsmittelphase unter Verwendung eines Mediums mit einer Oberflächenaktivität zwischen den beiden Arten von Lösungsmittelphasen durch Bewegen einer Mischung der ersten und zweiten Lösungsmittelphase, wobei die erste Lösungsmittelphase diejenige Lösungsmittelphase ist, in der das Proben-Biopolymer mit dem markierten Sonden-Biopolymer reagieren gelassen wurde; und
Nachweisen des in der zweiten Lösungsmittelphase enthaltenen Reaktionsprodukts unter Verwendung der Markierung,
wobei die erste Lösungsmittelphase eine wässrige Phase ist,
die zweite Lösungsmittelphase eine organische Phase ist,
das Proben-Biopolymer Polarität aufweist,
das Sonden-Biopolymer wenig Polarität aufweist, und
das Medium mit der Oberflächenaktivität einen Teil mit Polarität und einen Teil mit wenig Polarität enthält, wobei das Medium ein kationisches oberflächenaktives Mittel ist, und
wobei das Sonden-Biopolymer eines ist von: PNA (Peptid-Nukleinsäuren) und LNA (Locked-Nukleinsäuren) und wobei das Proben-Biopolymer DNA ist.

2. Verfahren zum Nachweisen eines Biopolymers nach Anspruch 1, unter Verwendung von zwei Arten von Lösungsmittelphasen, die hinsichtlich ihrer Eigenschaften unterschiedlich zueinander sind, wobei das Verfahren die Schritte umfasst:
Reagierenlassen des Proben-Biopolymers mit dem markierten Sonden-Biopolymer in der ersten Lösungsmittelphase;
Übertragen des Produkts der Reaktion des Proben-Biopolymers mit dem Sonden-Biopolymer in eine zweite Lösungsmittelphase unter Verwendung des Mediums mit einer Oberflächenaktivität zwischen den beiden Arten von Lösungsmittelphasen; und
Nachweisen des in der ersten Lösungsmittelphase enthaltenen Sonden-Biopolymers und des in der zweiten Lösungsmittelphase enthaltenen Reaktionsprodukts unter Verwendung der Markierung.

3. Verfahren zum Nachweisen eines Biopolymers nach Anspruch 2, wobei Reaktivität zwischen dem Proben-Biopolymer und dem Sonden-Biopolymer durch Vergleich einer nachgewiesenen Menge des in der ersten Lösungsmittelphase enthaltenen Sonden-Biopolymers mit einer nachgewiesenen Menge des in der zweiten Lösungsmittelphase enthaltenen Reaktionsprodukts bestimmt wird.

4. Verfahren zum Nachweisen eines Biopolymers nach einem der Ansprüche 1-3, wobei das Sonden-Biopolymer mit einem Partikel markiert ist, wobei das Partikel ein Halbleiter-Nanopartikel ist.

## Revendications

1. Procédé de détection d'un biopolymère utilisant deux types de phases de solvants présentant des propriétés qui diffèrent l'une de l'autre, le procédé comprenant les étapes consistant à :
permettre à un biopolymère échantillon de réagir avec un biopolymère sonde marqué dans une première phase de solvants ;
transférer un produit de la réaction du biopolymère échantillon avec le biopolymère sonde dans une seconde phase de solvants utilisant un milieu ayant une activité de surface située entre les deux types de phases de solvants en agitant un mélange de la première et de la seconde phase de solvants, ladite première phase de solvants étant la phase de solvants dans laquelle ledit biopolymère échantillon a été admis à réagir avec ledit biopolymère sonde marqué ; et
détecter le produit de la réaction contenu dans la seconde phase de solvants en utilisant le marqueur,
dans lequel :
la première phase de solvants est une phase aqueuse,
la seconde phase de solvants est une phase organique,
le biopolymère échantillon présente une polarité,
le biopolymère sonde présente une faible polarité, et
le milieu ayant l'activité de surface contient une partie présentant une polarité et une partie présentant une faible polarité, ledit milieu étant un tensioactif cationique, et
dans lequel le biopolymère sonde correspond à l'un quelconque parmi des PNA (acides nucléiques peptidiques) et des LNA (acides nucléiques verrouillés), et dans lequel le biopolymère échantillon est un ADN.

2. Procédé de détection d'un biopolymère selon la revendication 1, utilisant deux types de phases de solvants présentant des propriétés qui diffèrent l'une de l'autre, le procédé comprenant les étapes consistant à :
permettre audit biopolymère échantillon de réagir avec ledit biopolymère sonde marqué dans ladite première phase de solvants ;
transférer ledit produit de la réaction du biopolymère échantillon avec le biopolymère sonde dans ladite seconde phase de solvants utilisant ledit milieu ayant une activité de surface située entre les deux types de phases de solvants ; et
détecter le biopolymère sonde contenu dans la première phase de solvants, et le produit de la réaction contenu dans la seconde phase de solvants en utilisant le marqueur.

3. Procédé de détection d'un biopolymère selon la revendication 2, dans lequel :
la réactivité entre le biopolymère échantillon et le biopolymère sonde est déterminée en comparant une quantité détectée du biopolymère sonde contenu dans la première phase de solvants à une quantité détectée du produit de la réaction contenu dans la seconde phase de solvants.

4. Procédé de détection d'un biopolymère selon l'une quelconque des revendications 1 à 3, dans lequel le biopolymère sonde est marqué par une particule,
la particule étant une nanoparticule semiconductrice.
